(19) Europäisches Patentamt
European Patent Office
Office européen des brevets

(11) Veröffentlichungsnummer: **0 441 235 A2**

(12) # EUROPÄISCHE PATENTANMELDUNG

(21) Anmeldenummer: **91101176.5**

(51) Int. Cl.5: **C07D 209/48**

(22) Anmeldetag: **30.01.91**

(30) Priorität: **05.02.90 DE 4003309**

(43) Veröffentlichungstag der Anmeldung:
**14.08.91 Patentblatt 91/33**

(84) Benannte Vertragsstaaten:
**BE CH DE FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Fuchs, Hermann, Dr.**
**Altenhainer Strasse 2**
**W-6240 Königstein/Taunus(DE)**
Erfinder: **Gethöffer, Hanspeter, Dr.**
**Geisenheimer Strasse 97**
**W-6000 Frankfurt am Main(DE)**
Erfinder: **Gilb, Walter, Dr.**
**Frankfurter Strasse 58**
**W-6238 Hofheim am Taunus(DE)**

(54) **Verfahren zur kontinuierlichen Herstellung von Imidoperoxicarbonsäuren.**

(57) Verfahren zur kontinuierlichen Herstellung von Imidoperoxicarbonsäuren der Formel

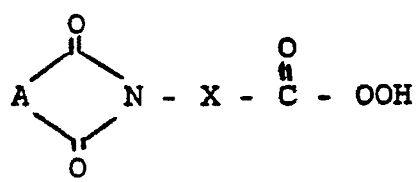

worin
A
$C_2$-$C_4$-Alkylen, Phenylen, Naphthylen oder eine Gruppe der Formel

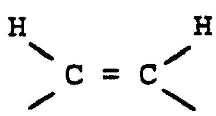

oder

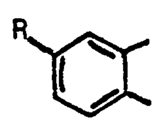

und R eine Gruppe der Formel -COOH, -$SO_3$H oder -Cl und
X
$C_1$-$C_{19}$-Alkylen oder Arylen bedeuten, wobei
man eine Lösung von Imidocarbonsäuren der Formel

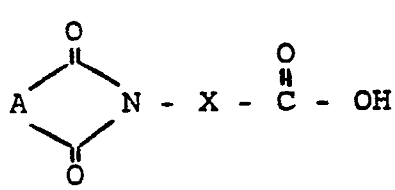

EP 0 441 235 A2

in Schwefelsäure oder Methansulfonsäure und eine wäßrige Lösung von Wasserstoffperoxid in einen statischen Mischer kontinuierlich eindosiert, anschließend die dabei erhaltene Lösung der Imidoperoxicarbonsäure mit Wasser versetzt und das ausgefallene Produkt isoliert.

## VERFAHREN ZUR KONTINUIERLICHEN HERSTELLUNG VON IMIDOPEROXICARBONSÄUREN

Imidoperoxicarbonsäuren können sowohl als Oxidationsmittel in der organischen Synthese (K. Balenkovic et al., J. Chem. Soc, 1962, 3821) als auch bei der Bleiche von Textilien eingesetzt werden, da ihre Wirkung schon bei 60° C und darunter auftritt (EP 325 289).

In den oben zitierten Veröffentlichungen werden zur Herstellung dieser Substanzen übliche, dem Stand der Technik entsprechende Verfahren angegeben, wie z.B. Lösen oder Suspendieren der Imidocarbonsäuren in Schwefelsäure oder Methansulfonsäure und anschließendes Versetzen mit konzentrierter wäßriger Wasserstoffperoxidlösung unter Kühlung. Aufgrund der möglichen Gefahr von Explosionen wurden diese Verfahren bisher nicht kontinuierlich durchgeführt. Kontinuierliche Verfahren zur Oxidation von Carbonsäuren sind bisher nur für aliphatische Carbonsäuren, wie Essigsäure oder 1,12-Dodekandicarbonsäure, bekannt.

So wird zur Oxidation der 1,12-Dodekandicarbonsäure in der US-PS 4 233 235 ein Verfahren beschrieben, bei dem die Reaktion in einem kontinuierlichen Rührreaktor oder einem Umlaufreaktor durchgeführt wird, wobei in letztem Falle eine Durchmischung mittels einer Pumpe erfolgt, in der die Edukte eingespeist werden. Ein Teil des ausgefallenen Produktes wird durch Filtration kontinuierlich aus dem Umlauf entfernt, der andere in die Pumpe zurückgeführt. Eine derartige Prozeßführung erfordert einen relativ großen apparativen und regeltechnischen Aufwand und birgt die Gefahr einer heftigen exothermen Zersetzung der im Kreis geführten Reaktionsmischung.

Überraschenderweise wurde nun ein Verfahren gefunden, welches in einem einfachen und kontinuierlichen Prozeß Imidoperoxicarbonsäuren in hoher Ausbeute und Reinheit liefert und die oben geschilderten Nachteile nicht aufweist.

Gegenstand der Erfindung ist deshalb ein Verfahren zur kontinuierlichen Herstellung von Imidoperoxicarbonsäuren der Formel

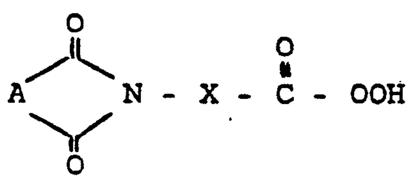

worin

A

$C_2$-$C_4$-Alkylen, Phenylen, Naphthylen oder eine Gruppe der Formel

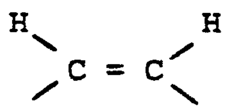

oder

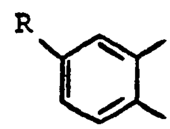

mit R eine Gruppe der Formel -COOH, $-SO_3H$ oder -Cl, bevorzugt -COOH und

X

$C_1$-$C_{19}$-Alkylen, bevorzugt $C_2$-$C_{11}$-Alkylen, oder Arylen, bevorzugt Phenylen, bedeuten, dadurch gekennzeichnet, daß man eine Lösung von Imidocarbonsäure der Formel

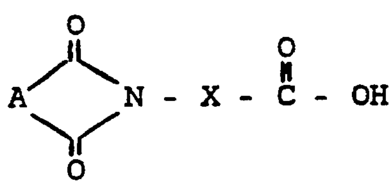

in Schwefelsäure oder Methansulfonsäure und eine wäßrige Lösung von Wasserstoffperoxid in einen statischen Mischer kontinuierlich eindosiert, anschließend die dabei erhaltene Lösung der Imidoperoxicarbonsäure mit Wasser versetzt und das ausgefallene Produkt isoliert. Statische Mischer enthalten bekanntlich keine rührenden Elemente. Die Durchmischung der Flüssigkeiten erfolgt gewöhnlich durch Verwirbeln an Schikanen, die im Innern des Mischers angeordnet sind.

Bei der Durchführung des Verfahrens erwies es sich als günstig, ein Gewichtsverhältnis von Schwefelsäure oder Methansulfonsäure zu Imidocarbonsäure von 1-10 zu 1, bevorzugt 2-4 zu 1 zu wählen. Im allgemeinen verwendet man 65-100 %ige, vorzugsweise 90-98 %ige Schwefelsäure, um zum einen die Imidocarbonsäuren in Lösung zu bringen und zum anderen die Imidocarbonsäuren für die Oxidationsreaktion vollständig zu protonieren. Ferner ist es sinnvoll, die Umsetzung mit einem Molverhältnis von Wasserstoffperoxid (100 %ig) zu Imidocarbonsäure von 1-4 zu 1, bevorzugt 1,5-3 zu 1 durchzuführen. Für das erfindungsgemäße Verfahren verwendet man handelsübliche wäßrige Wasserstoffperoxidlösungen, vorzugsweise 35-90 %ige Einstellungen.

Bei der Zusammenführung der Eduktströme ist darauf zu achten, daß Wasser und Schwefelsäure in einem solchen Verhältnis vorliegen, daß die Imidocarbonsäure bzw. Imidoperoxicarbonsäure nicht ausfällt und die Vorrichtung verstopft wird. Die beiden Lösungen der Ausgangsstoffe werden daher für die Reaktion aus entsprechenden Vorratsgefäßen kontinuierlich gefördert und in den statischen Mischer kontinuierlich eindosiert. Es erwies sich dabei als vorteilhaft, die Zuleitungen aus den Vorratsgefäßen in der Weise an den Mischer anzuschließen, daß das eine Rohr innerhalb des anderen Rohres verläuft, d.h. daß sie konzentrisch angeordnet sind. Die Dosierungsrate beträgt im allgemeinen bei der Imidocarbonsäurelösung 40-100 kg/Stunde, bevorzugt 50-80 kg/Stunde und bei der Lösung des Wasserstoffperoxids 8-20 kg/Stunde, bevorzugt 10-16 kg/Stunde. Beide Lösungen werden gleichzeitig in den Mischer eingeleitet.

Die Umsetzung in dem statischen Mischer wird bei der durch Hydratations- und Reaktionswärme hervorgerufenen Temperatur ohne Kühlung durchgeführt und liegt vorzugsweise zwischen 60 und 80 °C.

Der Mischer besteht aus einem Rohr mit einem relativ kleinen Volumen, wie es für eine relativ kurze Verweilzeit der Reaktionsmischung, vorzugsweise von 0,1-10 Sekunden, insbesondere 1-3 Sekunden notwendig ist.

Als statischer Mischer wird zum Beispiel ein SMX-Mischer der Fa. Sulzer verwendet. Der Mischer kann zudem für eine bessere Durchmischung der Reaktanten mit Füllkörpern bestückt werden. Diese Füllkörper sind jedoch für das erfindungsgemäße Verfahren nicht zwingend notwendig.

Es erwies sich ferner als besonders vorteilhaft, im direkten Anschluß an den Mischer und vor der Ableitung einen Reaktionsraum für eventuelle Nachreaktionen vorzusehen. Dieser Nachreaktionsraum ist normalerweise mit einer Kühlung versehen, so daß die Reaktionsmischung auf eine niedrigere Temperatur von 30-65 °C, vorzugsweise 40-60 °C abgekühlt wird. Der Nachreaktionsraum besteht zweckmäßigerweise aus einem Rohr mit einem so großen Volumen, wie für eine Verweilzeit der Reaktionsmischung von 3-300 Sekunden, vorzugsweise 30-90 Sekunden notwendig ist.

Die Reaktionsmischung wird nach Verlassen des stat. Mischers bzw. Nachreaktionsraumes über einen zweiten Mischer mit Wasser versetzt und die ausgefallene Imidoperoxicarbonsäure isoliert. Es ist auch möglich, die Reaktionsmischung über eine Ableitung in Eiswasser zu leiten und so die Imidoperoxicarbonsäure auszufällen. Üblicherweise wird die ausgefallene Imidoperoxicarbonsäure aus der Reaktionsmischung abfiltriert, mineralsäurefrei gewaschen und getrocknet.

Im allgemeinen können Peroxicarbonsäuren auch durch Phlegmatisierungsmittel stabilisiert werden. Als Phlegmatisierungsmittel kommen Alkali-, Erdalkali- oder Erdmetallsulfate oder Borsäure in Frage. Diese Stoffe können sowohl in fester Form oder als wäßrige Lösungen bzw. Suspensionen nach der Umsetzung in Mengen von 0 bis 80 Gew.-%, bezogen auf die Peroxicarbonsäure zugesetzt werden. Es ist auch möglich die Schwefelsäure durch Zusatz von Alkalihydroxiden nur zum Teil zu neutralisieren und das entstandene Alkalisulfat als Phlegmatisierungsmittel zu verwenden (in situ - Phlegmatisierung). Wurde eine in situ-Phlegmatisierung durchgeführt, so entfällt der Waschvorgang vor der Trocknung.

Der Vorteil des erfindungsgemäßen Verfahrens liegt darin, daß in einem kontinuierlichen Verfahren die Imidocarbonsäure in konzentrierter Schwefelsäure gefahrlos mit einer konzentrierten Wasserstoffperoxidlösung vermischt und zur Reaktion gebracht werden kann. Dabei ist die Verweilzeit der Reaktionsteilnehmer in der Mischvorrichtung relativ kurz, so daß keine externe Kühlung notwendig ist. Es ist günstig, die Oxidationsreaktion der Imidocarbonsäure mit Wasserstoffperoxid in der Mischvorrichtung bei der durch die Hydratations- und Reaktionswärme hervorgerufenen Wärmetönung durchzuführen. Überraschenderweise wird dabei innerhalb der kurzen Kontaktzeiten ein vollständiger Umsatz gefahrlos gewährleistet.

Die nach dem beanspruchten Verfahren hergestellten Imidoperoxicarbonsäuren werden im allgemeinen als Oxidationsmittel in der organischen Synthese oder als Bleichmittel in Waschmittelformulierungen für Temperaturen unter 60 °C eingesetzt.

**Beispiel:**

Herstellung von ε-Phthalimidoperoxicapronsäure

18,75 kg einer Lösung von 29,7 % ε-Phthalimidocapronsäure in konzentrierter Schwefelsäure werden mit 75 kg/Stunde und 3,7 kg einer 50 %igen wäßrigen Wasserstoffperoxidlösung mit 14,8 kg/Stunde gleichzeitig in einen statischen rohrförmigen Mischer (Ø = 15 mm, l = 80 mm) kontinuierlich eindosiert. Danach durchläuft die Reaktionsmischung einen rohrförmigen Nachreaktionsraum (Ø = 15 mm, l = 3000 mm) mit einer Kühlwasser-Mantelkühlung und wird anschließend in ein gerührtes Vorlagegefäß (100 l Kessel aus Stahl/Email) eingeleitet. Am Ausgang des statischen Mischers beträgt die Temperatur der Reaktionsmischung 60 °C und wird durch die externe Kühlung des Nachreaktionsraumes auf 40 °C abgekühlt. Beim Einleiten der Reaktionsmischung in das Vorlagegefäß, welches mit einer Mischung aus 20 kg Wasser und 20 kg Eis gefüllt ist, fällt die ε-Phthalimidoperoxicapronsäure als Feststoff aus. Das ausgefallene Produkt wird in einer Filterpresse (Inhalt 24 l) isoliert und mit Wasser sulfatfrei gewaschen. Nach zehnstündigem Trockenblasen erhält man 11,5 kg Feststoff mit einem Wassergehalt von 50 % (bestimmt nach der Karl-Fischer Methode) und einem Wirkstoffgehalt von 45,4 % (entspricht 91 % Umsatz, Bestimmung des Aktivsauerstoffgehaltes durch iodometrische Titration).

**Patentansprüche**

**1.** Verfahren zur kontinuierlichen Herstellung von Imidoperoxicarbonsäuren der Formel

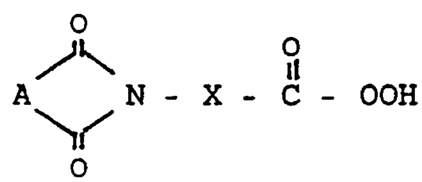

worin

A

$C_2$-$C_4$-Alkylen, Phenylen, Naphthylen oder eine Gruppe der Formel

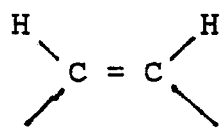

oder

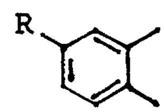

mit R eine Gruppe der Formel -COOH, -$SO_3$H oder -Cl, bevorzugt -COOH und

X

$C_1$-$C_{19}$-Alkylen, bevorzugt $C_2$-$C_{11}$-Alkylen, oder Arylen, bevorzugt Phenylen, bedeuten, dadurch gekennzeichnet, daß man eine Lösung von Imidocarbonsäure der Formel

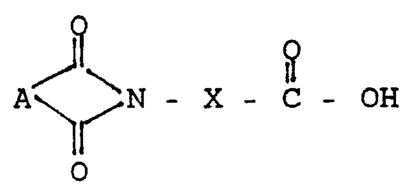

in Schwefelsäure oder Methansulfonsäure und eine wäßrige Lösung von Wasserstoffperoxid in einen statischen Mischer kontinuierlich eindosiert, anschließend die dabei erhaltene Lösung der Imidoperoxicarbonsäure mit Wasser versetzt und das ausgefallene Produkt isoliert.

**2.** Verfahren nach Anspruch 1, dadurch gekennzeichnet, daß die Verweilzeit der Imidocarbonsäurelösung und der Wasserstoffperoxidlösung im statischen Mischer 0,1 bis 10 Sekunden beträgt.

**3.** Verfahren nach Anspruch 1 oder 2, dadurch gekennzeichnet, daß die Imidocarbonsäurelösung und die Wasserstoffperoxidlösung über zwei konzentrische Rohre in den statischen Mischer eindosiert werden.

4. Verfahren nach einem der Ansprüche 1 bis 3, dadurch gekennzeichnet, daß die Dosierungsrate der Imidocarbonsäurelösung 40-100 kg/Stunde und die Dosierungsrate der Wasserstoffperoxidlösung 8-20 kg/Stunde beträgt.

5. Verfahren nach einem der Ansprüche 1 bis 4, dadurch gekennzeichnet, daß die Dosierungsrate der Imidocarbonsäure 50-80 kg/Stunde und die Dosierungsrate der Wasserstoffperoxidlösung 10-16 kg/Stunde beträgt.

6. Verfahren nach einem der Ansprüche 1 bis 5, dadurch gekennzeichnet, daß das Reaktionsgemisch einen kühlbaren Nachreaktionsraum durchläuft, wobei das Reaktionsgemisch auf eine Temperatur von vorzugsweise 30 bis 65 °C gekühlt wird.

7. Verfahren nach einem der Ansprüche 1 bis 6, dadurch gekennzeichnet, daß das Gewichtsverhältnis von Schwefelsäure oder Methansulfonsäure zu Imidocarbonsäure 1 bis 10 zu 1 beträgt.

8. Verfahren nach einem der Ansprüche 1 bis 7, dadurch gekennzeichnet, daß die Konzentration der Schwefelsäure 65 bis 100 Gew.-% beträgt.

9. Verfahren nach einem der Ansprüche 1 bis 8, dadurch gekennzeichnet, daß das Molverhältnis von Wasserstoffperoxid (100 %ig) zu Imidocarbonsäure 1 bis 4 zu 1 beträgt.

10. Verfahren nach einem der Ansprüche 1 bis 9, dadurch gekennzeichnet, daß die Reaktion im statischen Mischer ohne Kühlung bei einer Reaktionstemperatur von 60 bis 80 °C durchgeführt wird.